# EUROPÄISCHE PATENTSCHRIFT

(11) **EP 1 898 696 B1**
(45) Veröffentlichungstag und Bekanntmachung des Hinweises auf die Patenterteilung: **16.05.2012**
(21) Anmeldenummer: 06761993.2
(22) Anmeldetag: 08.06.2006
(51) Int. Cl.: A01N 65/26

(54) **VERFAHREN ZUR HERSTELLUNG EINES MARGOSAEXTRAKTES**
METHOD FOR PRODUCING MARGOSA EXTRACT
PROCEDE POUR PRODUIRE UN EXTRAIT DE MARGOUSIER

(30) Priorität: 08.06.2005 DE 102005026424
(43) Veröffentlichungstag der Anmeldung: 19.03.2008
(73) Patentinhaber: Terra Nostra Produkte mit Naturextrakten GmbH, 85290 Geisenfeld (DE); Rembold, Heinz, 81379 München (DE)
(72) Erfinder: REMBOLD, Heinz, 81379 München (DE)
(74) Vertreter: Rembold, Hansjörg
(86) Internationale Anmeldenummer: PCT/EP2006/005505
(87) Internationale Veröffentlichungsnummer: WO 2006/131378

(56) Entgegenhaltungen:
- WO-A-97/25867
- US-A- 4 679 737
- US-A- 5 229 007
- US-A- 5 372 817
- US-A- 5 885 600
- US-B2- 6 855 351
- REMBOLD H ET AL: "Control of the house dust mite, Dermatophagoides farinae, by biologically active seed components of the neem tree, Azadirachta indica A. Juss" ALLERGO JOURNAL, MUENCHEN, DE, Bd. 13, Nr. 4, 2004, Seiten 269-273, XP008082812 ISSN: 0941-8849 in der Anmeldung erwähnt
- SINNIAH D ET AL: "Reye-Like Syndrome Due to Margosa Oil Poisoning : Report of Case with Postmortem Findings" AMERICAN JOURNAL OF GASTROENTEROLOGY, NEW YORK, NY, US, Bd. 77, Nr. 3, 1982, Seiten 158-161, XP008082805 ISSN: 0002-9270

## Beschreibung

Die Erfindung betrifft ein Verfahren zur Herstellung eines Margosaextraktes, einen Margosaextrakt, wie er mit dem erfindungsgemäßen Verfahren erhalten wird, die Verwendung dieses Margosaextraktes sowie ein Schädlingsbekämpfungsmittel, welches den Margosaextrakt enthält.

Der Neembaum (*Azadirachta indica*) und besonders dessen Samen sind in jüngerer Zeit auf dem Gebiet der Schädlingsbekämpfung in das wissenschaftliche Interesse gerückt. Die vielfältigen Möglichkeiten zur Verwendung des zur Familie der Mahagonibäume gehörenden, etwa 15 Meter hoch wachsenden und bis zu hundert Kilogramm Früchte liefernden Baumes sind in H. Schmutterer, The Neem Tree, Neem Foundation, Mumbai 2002, 2nd Edition, ausführlich beschrieben. Das aus Neemsamen gewonnene Neemöl ist in der EU mit der CAS Nummer 11141-17-6 und als Margosaextrakt unter der vorläufigen Notifizierungsnummer [N621] registriert.

Der Neembaum und seine Bestandteile sind fester Bestandteil der indischen Naturmedizin. Gemahlene Neemnüsse, aus Neemnüssen gepresstes Neemöl und der beim Pressen zurückbleibende Presskuchen werden als Ausgangsstoffe für die Herstellung von höherwertigen Produkten, wie Seifen oder Zahnpasten oder auch direkt als Arzneimittel verwendet. Um dieses Wissen jedoch auch zum Nutzen von Verbrauchern in den Industriestaaten zur Anwendung bringen zu können, müssen die Produkte an die Ansprüche der Verbraucher angepasst werden. So enthalten Produkte, die durch Auspressen der Neemsamen gewonnen werden, also das Neemöl und der Presskuchen, Verbindungen, die bei Zutritt von Luft einen knoblauchartigen Geruch verursachen. Weiter weisen die aus Bestandteilen des Neembaums erhaltenen Produkte eine dunkle braune Färbung auf, die bei längerer Lagerung, insbesondere unter Luftzutritt, noch intensiver wird. Eine Bleichung ist nur für Anwendungen möglich, in denen die medizinische Wirkung der Inhaltsstoffe des Neemsamens nicht von Bedeutung ist, beispielsweise bei der Herstellung von Seifen. Sollen jedoch vorteilhafte Wirkungen des Neemsamens genutzt werden, die auf empfindliche Inhaltsstoffe des Neemsamens zurückgehen, beispielsweise Azadirachtin, ist eine Bleichung nur unter extrem milden Bedingungen möglich, die nicht zu einer Zerstörung der Inhaltsstoffe führt.

In der WO 97/25867 wird ein Verfahren zur Extraktion von Inhaltsstoffen aus Bestandteilen des Neembaums, vorzugsweise aus Samen und Nüssen beschrieben. Als Extraktionsmittel wird Kohlendioxid in subkritischem oder superkritischem Zustand verwendet, wobei das Extraktionsmittel im Kreislauf geführt wird. Dabei wird der gemahlene Neemsamen oder ein durch Auspressen der Neemsamen zur teilweisen Entfernung der öligen Bestandteile gewonnener Presskuchen verwendet. Der Extrakt zeigt hervorragende Eigenschaften als Mittel zur Bekämpfung von Schädlingen, insbesondere als Repellens gegen die Hausstaubmilbe.

In einer vergleichenden Studie (Allergo J. 2004, 13: 269-73) weisen H. Rembold und H. Oetzel nach, dass Formulierungen auf der Basis des durch CO₂-Extraktion gewonnenen Neemöls im Vergleich mit den auf der Basis von Pressöl hergestellten Produkten in ihrer das Milbenwachstum störenden Wirkung bis zu hundertfach wirksamer gegen die Hausstaubmilbe, *Dermatophagoides farinae,* sind.

US 6,855,351 B2 beschreibt die Herstellung einer lagerfähigen konzentrierten Emulsion auf der Basis von Neemöl, welches als Pestizid verwendet werden kann. Dazu wird zunächst Neemöl aus Samen des Neembaums gewonnen. Das Öl wird thermisch behandelt um seine Dichte zu erniedrigen und dann emulgiert. Ferner wird durch Extraktion mit einem Lösungsmittel aus Neemsamen ein konzentrierter Azadirachtinextrakt hergestellt, welcher zumindest 300 ppm Azadirachtin enthält. Das emulgierte Neemöl wird dann mit dem Azadirachtinextract vermischt wobei ein Pestizid erhalten wird, welches zumindest 300 ppm Azadirachtin enthält.

In der US 5,229,007 wird ein Verfahren beschreiben, welches es ermöglicht, selektiv Verunreinigungen, insbesondere Aflatoxine, aus einem azadirachtinhaltigen Material zu entfernen. Dazu wird das Material mit einem Bindemittel behandelt, welches selektiv die Verunreinigungen binden kann. Geeignet ist beispielsweise Aktivkohle.

In der US 5,885,600 wird eine Zusammensetzung mit insektenrepellierender Wirkung beschrieben, welche ein kaltgepresstes Öl und ein Antioxidationsmittel enthält, insbesondere eine Kombination von Neemöl, Citronellöl und Zedernöl.

In der US 5,372,817 wird ein aus Neemsamen hergestelltes Insektizid beschrieben. Dabei werden Neemsamen mit einem unpolaren Lösungsmittel extrahiert und der Extrakt nach Entfernen des Lösungsmittels gekühlt, sodass sich eine wachsartige feste Phase aus dem Öl abscheidet.

Sinniah et al: "Reye-like Syndrome due to Margosa Oil Poisoning: Report of Case with Postmortem Findings" American Journal of Gastroenterology, New York, NY, US, Bd. 77, Nr.3, 1982, Seiten 158 - 161, erläutert, dass die durch Vergiftung mit Margosaöl hervorgerufene, dem Reye-Syndrom ähnliche Symptome wahrscheinlich auf eine durch toxische Komponenten des Öls synergistisch verstärkte Wirkung von Verunreinigungen durch Aflatoxin zurückzuführen sind.

In der US 4,679,737 wird eine Mühle zur Gewinnung von gemahlenen, analytisch repräsentativen Proben aus einer größeren Charge entnommenen Probe, sodass auch unregelmäßig in der Charge verteilte hochgiftige Verunreinigungen erfasst werden können, beispielsweise Aflatoxine in Mais.

Versuche, die Extraktion der Neemsamen mit Kohlendioxid direkt am Ort der Erzeuger in Indien durchzuführen, schlugen bisher fehl. Trotz umfangreicher Bemühungen war es nicht möglich, eine gleichbleibende Qualität des Extraktes sicherzustellen. Man ist daher immer noch gezwungen, die Neemsamen aus dem Erzeugerland in ein Industrieland, wie Deutschland, zu importieren, um die gemahlenen Neemsamen dann dort mit Kohlendioxid zu extrahieren. Nur auf diese Weise konnte eine gleichbleibende Qualität des Extraktes erreicht werden. Bei den in Deutschland hergestellten Extrakten fielen jedoch immer wieder Chargen an, die einen unzulässig hohen Gehalt an Aflatoxinen aufweisen und daher verworfen werden mussten.

Der Gewinnung von Wirkstoffen aus natürlichem Material geht oft eine Vorlaufzeit voraus für das Sammeln und Trocknen, den Transport, und schließlich bei der Lagerung vor Ort bis zur endgültigen Verarbeitung. Bei höheren Temperaturen und höherer Feuchtigkeit, und vor allem auch ohne einen, oft unerwünschten, Einsatz von Fungiziden, ist die Gefahr des Pilzwachstums bei ölhaltigem Material besonders hoch, was dann zur Belastung des Guts mit Pilzgiften (Mykotoxinen) führen kann. Dabei stellen die Krebs erzeugenden Aflatoxine eine besondere Gefahr dar.

Pilze, vor allem der die kanzerogenen Aflatoxine produzierende *Aspergillus flavus,* stellen bei der Verarbeitung ölhaltiger Samen und damit auch von Neemsamen ein schwer zu kalkulierendes Risiko dar. Der Neembaum wächst nur in einem tropischen, feuchtwarmen Klima. Die Früchte müssen deshalb sofort nach der Ernte vom Fruchtfleisch befreit und die Nüsse im Schatten getrocknet werden, um eine Verminderung der biologisch aktiven Wirkstoffe zu vermeiden. Erst bei einem Wassergehalt von etwa 15% können dann die Nüsse in Containern verschickt werden die, falls sie nicht klimatisiert sind, beim Seetransport stark wechselnden Temperaturen ausgesetzt sind. Es besteht dann selbst bei einem vorher nicht verpilzten Material die Gefahr, dass sich feuchte, ein Pilzwachstum begünstigende Nischen bilden, die auch bei einer späteren fachgemäßen sichtkontrolle nicht unbedingt nachzuweisen sind. Die Sichtkontrolle erfolgt, indem aus einer bestimmten Anzahl der angelieferten Säcke Samen entnommen werden und durch Besichtigung auf eine Verpilzung überprüft werden. Dabei können jedoch jeweils nur eine begrenzte Anzahl von Säcken und bei den ausgewählten Säcken nur an einer bestimmten Stelle des Sackes Nüsse entnommen werden. Eine Überprüfung aller angelieferten Nüsse ist daher nicht möglich. Ob in einem in handliche Säcke unterteilten Material zufällig Aflatoxine enthalten sind, lässt sich deshalb oft erst nach der Aufarbeitung, also im fertigen Extrakt eindeutig feststellen.

Der durch Extraktion mit Kohlendioxid gewonnene Extrakt wird erfolgreich als Repellens gegen die Hausstaubmilbe eingesetzt. Diese nistet sich vor allem in Matratzen und Bettwäsche ein. Ihr Kot zeigt eine stark allergene Wirkung. Das Krankheitsbild wird auch als Hausstauballergie bezeichnet. Durch Applizieren des Neemextrakts in einer geeigneten Formulierung auf Matratzen und Bettwäsche kann eine signifikante Reduzierung der Hausstaübmilbenpopulation und damit der allergenen Belastung erreicht werden. Da der Extrakt jedoch großflächig über beispielsweise die Bettwäsche mit dem Menschen in Kontakt gelangen kann, muss sichergestellt werden, dass der Extrakt nicht mit Mykotoxinen, insbesondere Aflatoxinen belastet ist. Andererseits legen die Verbraucher großen Wert auf einen rein biologischen Ursprung des Extraktes, sodass auch keine synthetischen Konservierungsmittel verwendet werden können, um die Samen während des Transports vor einem Pilzbefall zu schützen.

Der Erfindung lag daher die Aufgabe zugrunde, ein Verfahren zur Herstellung eines Margosaextraktes zur Verfügung zu stellen, welches zu einem Extrakt führt, der zumindest die gleiche Aktivität aufweist, wie ein nach bisher bekannten Verfahren aus Neemsamen oder Presskuchen gewonnener Extrakt, wobei die Gefahr einer Verunreinigung des Extraktes mit Mykotoxinen, insbesondere Aflatoxinen weitgehend ausgeschlossen ist.

Diese Aufgabe wird mit einem Verfahren mit den Merkmalen des Patentanspruchs 1 gelöst. Vorteilhafte Weiterbildungen des Verfahrens sind Gegenstand der abhängigen Ansprüche.

Beim erfindungsgemäßen Verfahren wird der Extrakt durch Extraktion eines Öls mit Kohlendioxid gewonnen, wobei das Öl durch Pressen aus Neemsamen erhalten wird. Dies bietet den Vorteil, dass aus dem vereinigten Öl einer Charge Proben entnommen werden können, um auf eine Verunreinigung durch Aflatoxine überprüft zu werden. Es kann also der Aflatoxingehalt einer gesamten Charge mit einer einzelnen Messung überprüft werden und nicht lediglich stichprobenartig einzelne Nüsse. Dadurch kann bereits vor der Extraktion des durch Kaltpressen von Neemnüssen oder Neemsamen gewonnen Öls sichergestellt werden, dass der Extrakt nicht mit Mykotoxinen verunreinigt ist. Ferner benötigt das Öl für seinen Transport im Vergleich zu den Nüssen ein wesentlich kleineres Volumen, sodass die Transportkosten sinken. Das Öl ist auch wesentlich weniger empfindlich gegen einen Befall mit Pilzen, als dies bei den Neemnüssen der Fall ist. Dadurch sinkt auch das Transportrisiko. Ferner lässt sich das Auspressen auch zuverlässig im Ursprungsland der Neemnüsse bzw. -samen durchführen, sodass sich einerseits die Herstellung des Extrakts wesentlich verbilligen lässt und andererseits ein weiteres Glied der Wertschöpfungskette im Ursprungsland der Neemnüsse verbleibt, wodurch dort Arbeitsplätze entstehen bzw. zumindest gesichert werden können.

Die Extraktion mit überkritischem Kohlendioxid stellt bei der Gewinnung des Öls den größten Kostenfaktor dar. Mit dem hier gezeigten Verfahren wird ein Weg vorgestellt, der die Gewinnung von mykotoxinfreiem und dabei biologisch hoch wirksamem Neemöl, dem so genannten Margosaextrakt, aus einem nicht mit Mykotoxinen belasteten Öl ermöglicht.

Überraschend hat sich gezeigt, dass auch aus dem durch Pressen aus Neemsamen bzw. -Kernen gewonnenen Neemöl ein Extrakt gewonnen werden kann, der eine zumindest gleich hohe Aktivität zeigt wie ein Extrakt, der durch direkte Extraktion der Samen bzw. des Presskuchens gewonnen wird. Wie in dem oben genannten Artikel von Rembold und Oetzel gezeigt wurde, zeigt ein Extrakt, der direkt aus Neemsamen durch Extraktion mit Kohlendioxid gewonnen wurde, eine deutlich höhere Aktivität als das Pressöl. Während mit Proben, die Pressöl enthielten, in einem Langzeitversuch die Anzahl der lebenden Milben auf 576 bzw. 533 abgesenkt werden konnte, konnte mit Präparaten, die einen Extrakt enthielten, der durch Extraktion der Neemsamen mit Kohlendioxid gewonnen wurde, die Zahl der Milben auf 126 bzw. 28 abgesenkt werden. Die Zahl der Milben in einem Kontrollversuch, bei welchem kein Neemextrakt eingesetzt worden war, betrug 3.550. Bisher war man daher davon ausgegangen, dass ein hoher Wirkstoffgehalt nur dann erhalten werden kann, wenn die Extraktion direkt aus den gemahlenen Samen bzw. dem Presskuchen erfolgt. Man nimmt an, dass sich bei der Extraktion des Pressöls mit Kohlendioxid die Stoffe im Rückstand anreichern, welche das Wachstum von Hausstaubmilben nicht verhindern, sondern eher verstärken. Das raffinierte Neemöl hat dagegen einen stärker schädigenden Einfluss auf das Milbenwachstum als das Ausgangsmaterial. Beim erfindungsgemäßen Verfahren werden also vermutlich solche Stoffe abgetrennt, welche die Wirkung des Neemöls auf die Milben verringern.

Das als Ausgangsmaterial verwendete Pressöl kann durch Pressen in üblichen Ölmühlen aus den Neemsamen bzw. -kernen gewonnen werden. Die Neemsamen bzw. -kerne werden vorzugsweise kaltgepresst.

Bei der nachfolgenden Extraktion des Pressöls mit Kohlendioxid wird in hoher Ausbeute ein klares gelbes Öl erhalten, das praktisch geruchsfrei ist und auch bei längerer Lagerung an Luft keinen ranzigen Geruch entwickelt.

Zur einfacheren Extraktion wird das Pressöl vorzugsweise auf einen inerten Träger aufgebracht. Dies kann beispielsweise erfolgen, indem das Öl mit dem Träger vermischt wird, wobei die Menge des Trägers vorzugsweise so gewählt wird, dass ein frei fließendes Pulver erhalten wird. Als Träger werden bevorzugt anorganische Träger verwendet. Nach der Extraktion kann der mit Rückständen belegte Träger beispielsweise als Düngemittel oder als Futterzuschlagstoff eine weitere Anwendung finden. Der Träger liegt daher bevorzugt in Pulverform vor. Geeignete anorganische Materialien sind beispielsweise Kieselgur und Kieselgel. Es kann jedoch auch ein grobteiligeres Material eingesetzt werden, wie beispielsweise Tongranulate oder gemahlener Bimsstein. Das Verhältnis von Trägermaterial und Pressöl wird vorzugsweise so gewählt, dass, wie bereits erwähnt, ein freifließendes Pulver erhalten wird, das einen möglichst hohen Gehalt an Öl aufweist. Das Verhältnis von Träger zu Öl wird bevorzugt im Bereich von 3 : 1 bis 1 : 2, vorzugsweise 2 : 1 bis 1 : 1 gewählt.

Die Extraktion des Öls wird in üblichen Extraktionsanlagen durchgeführt. Die Extraktionsbedingungen werden so gewählt, dass die Kohlendioxidextraktion bei einem Druck von 250 bis 600 bar, vorzugsweise 300 bis 400 bar durchgeführt wird. Weiterhin wird die Extraktion bevorzugt bei einer Temperatur zwischen 30 und 70 °C, vorzugsweise zwischen 30 und 45 °C durchgeführt. Bei dieser Temperatur besitzt das Kohlendioxid eine gute Extraktionswirkung, insbesondere für Azadirachtine. Ferner kann die Extraktion bei diesen Temperaturen so schonend durchgeführt werden, dass kein Aktivitätsverlust des Extraktes in Kauf genommen werden muss.

Besonders bevorzugt wird das Extraktionsmittel im Kreislauf geführt. Für die Extraktion sind daher vergleichsweise geringe Mengen an Kohlendioxid erforderlich. Beim erfindungsgemäßen Verfahren werden sehr hohe Ausbeuten erhalten. Die Störstoffe bleiben im Extraktionsgefäß zurück, beispielsweise gebunden an den inerten Träger. Auch bei längerer Extraktionsdauer, beispielsweise bei größeren Chargen, wird keine Verschlechterung der Aktivität des Extraktes beobachtet, d.h. die Störstoffe bleiben fest am inerten Träger gebunden und werden durch das im Kreis geführte Kohlendioxid nicht in den Extrakt verschleppt. Bevorzugt wird die Extraktion jedoch in der Weise geführt, dass die Extraktion abgebrochen wird, wenn 90-95 % des eingesetzten Neemöls in der Vorlage als Extrakt erhalten sind. In den restlichen 5 % des eingesetzten Öls aus Kernen und/oder Samen des Neembaums sind schwerer lösliche Störkomponenten, wie Farbstoffe, oxidierte Ölbestandteile und andere unerwünschte Komponenten in angereicherter Form enthalten, aber praktisch keine aktiven Inhaltsstoffe. Man kann dadurch die Reinheit und damit die Qualität des erhaltenen Margosaextrakts weiter steigern.

Das Extraktionsmittel wird vorzugsweise bei einer Temperatur von 10 bis 80 °C, vorzugsweise 25 bis 40 °C auf einen Druck von 30 bis 80 bar, vorzugsweise 50 bis 70 bar entspannt. Auf diese weise wird eine thermische Belastung der im Extrakt enthaltenen Stoffe vermieden, die zu einer Zersetzung und damit einem Aktivitätsverlust führen könnte.

Dem Extraktionsmittel, im Wesentlichen Kohlendioxid, kann ein Schleppmittel beigegeben sein. Das Schleppmittel weist vorzugsweise eine höhere Polarität auf als Kohlendioxid. Geeignete Schleppmittel sind vorzugsweise ausgewählt aus der Gruppe von Alkoholen, Ketonen, Estern, Nitrilen oder zyklischen Ethern. Insbesondere um nach der Extraktion eine leichte Entfernung des Schleppmittels zu ermöglichen, werden bevorzugt Schleppmittel verwendet, die eine Kohlenstoffanzahl zwischen 1 und 5 aufweisen. Die Schleppmittel besitzen dann einen ausreichend niedrigen Siedepunkt, sodass sie sich leicht unter vermindertem Druck aus dem Extrakt entfernen lassen.

Der Anteil des Schleppmittels am Extraktionsmittel beträgt vorzugsweise 0,5 bis 30 %, insbesondere bevorzugt 2 bis 10 %.

Vorzugsweise erfolgt die Extraktion mit reinem Kohlendioxid.

Die Extraktion des Pressöls kann fraktioniert durchgeführt werden. Dadurch kann ggf. eine Abtrennung störender Limonoide erreicht werden. Die Fraktionierung des Pressöls kann erfolgen, indem beispielsweise die Extraktion bei unterschiedlichen Drücken durchgeführt wird, der Druck während der Extraktion also beispielsweise schrittweise erhöht wird. Eine Fraktionierung kann auch dadurch erreicht werden, dass die Konzentration eines zugesetzten Schleppmittels verändert wird, beispielsweise schrittweise erhöht wird. Eine solche Fraktionierung ist beispielsweise sinnvoll, um Fraktionen mit einem hohen Azadirachtingehalt zu erhalten. Beim erfindungsgemäßen Verfahren ist eine Fraktionierung jedoch nur in Ausnahmefällen erforderlich. Durch das Abpressen scheint ein erheblicher Anteil der Störstoffe im Presskuchen zu verbleiben, sodass spezielle Fraktionierungsverfahren an sich nicht erforderlich sind.

Die Dauer des Extraktionsvorgangs richtet sich nach der Ansatzgröße und kann vom Fachmann leicht ermittelt werden, indem beispielsweise die Menge des Extrakts pro Zeiteinheit gemessen wird. Dies ist sehr einfach dadurch möglich, dass die zuvor gewogene Vorlage regelmäßig ausgetauscht und die Menge des in der Vorlage angesammelten Extrakts durch Differenzwägung bestimmt wird. Üblicherweise wird für die Extraktion von 100 kg Pressöl eine Extraktionsdauer von etwa 2 bis 9 Stunden, vorzugsweise 3 bis 6 Stunden benötigt.

Mit dem erfindungsgemäßen Extraktionsverfahren wird ein Margosaextrakt erhalten, der ein hellgelbes Öl darstellt und der beispielsweise eine sehr hohe repellierende Wirkung gegenüber der Hausstaubmilbe zeigt. Gegenstand der Erfindung ist daher auch ein Margosaextrakt, wie er mit dem oben beschriebenen Verfahren erhalten werden kann. Der erfindungsgemäße Margosaextrakt zeichnet sich durch charakteristische Merkmale im UV-Spektrum sowie bei den relativen Intensitäten eines Gaschromatogramms aus.

Der erfindungsgemäße Margosaextrakt zeigt eine sehr gute Wirkung als Mittel zur Schädlingsbekämpfung. Gegenstand der Erfindung ist daher auch die Verwendung des oben beschriebenen Margosaextrakt als Schädlingsbekämpfungsmittel. Eine besonders gute Wirkung wird beobachtet, wenn der Margosaextrakt als Repellens für Hausstaubmilben verwendet wird. Der Margosaextrakt wirkt dabei nicht als Akarizid, tötet die Milben also nicht direkt ab. Vielmehr scheint die Wirkung des Extraktes darin zu bestehen, dass die Milben die Nahrungsaufnahme verweigern, also verhungern. Dadurch verringert sich auch die Menge des ausgeschiedenen Milbenkots und damit die Menge des Allergens, das beispielsweise in einer Matratze oder einem Oberbett enthalten ist. Die Wirkung des Margosaextrakts auf die Hausstaubmilbe lässt sich deshalb direkt durch deren Kotausscheidung quantifizieren.

Ein weiterer Gegenstand der Erfindung ist ein Schädlingsbekämpfungsmittel, welches den oben beschriebenen Margosaextrakt enthält. Dazu kann beispielsweise der Extrakt in einem geeigneten Lösungsmittel, beispielsweise einem Alkohol oder Alkohol/Wassergemisch aufgenommen werden. Werden Alkohol/Wassergemische verwendet, wird der Wassergehalt bevorzugt kleiner als 30 Gew. % gewählt. Dabei sind vergleichsweise niedrige Konzentrationen des Extraktes im Schädlingsbekämpfungsmittel erforderlich. Geeignet wird die Konzentration des Margosaextrakts im Lösungsmittel in einem Bereich von 0,1 bis 10 Gew. %, vorzugsweise 0,5 bis 5 Gew. %, insbesondere bevorzugt 0,6 bis 1 Gew. % gewählt. Der Extrakt lässt sich dadurch in sehr verdünnter Form z.B. auf einer Matratze oder in einer Füllung einer Matratze oder eines Oberbetts verteilen.

Die Erfindung wird im Weiteren unter Bezugnahme auf die beigefügten Figuren näher erläutert. Dabei zeigt:
- Fig. 1:: Eine schematische Darstellung der Vorrichtung zur Durchführung des erfindungsgemäßen Verfahrens;
- Fig. 2:: ein HPLC-Diagramm der Fraktionen A und B;
- Fig. 3:: ein UV-Spektrum des als Ausgangsmaterial verwendeten Pressöls sowie der extrahierten Fraktionen A und B;
- Fig. 4:: eine Abbildung von Testblättchen, auf welchen Milbenkot abgelagert ist.

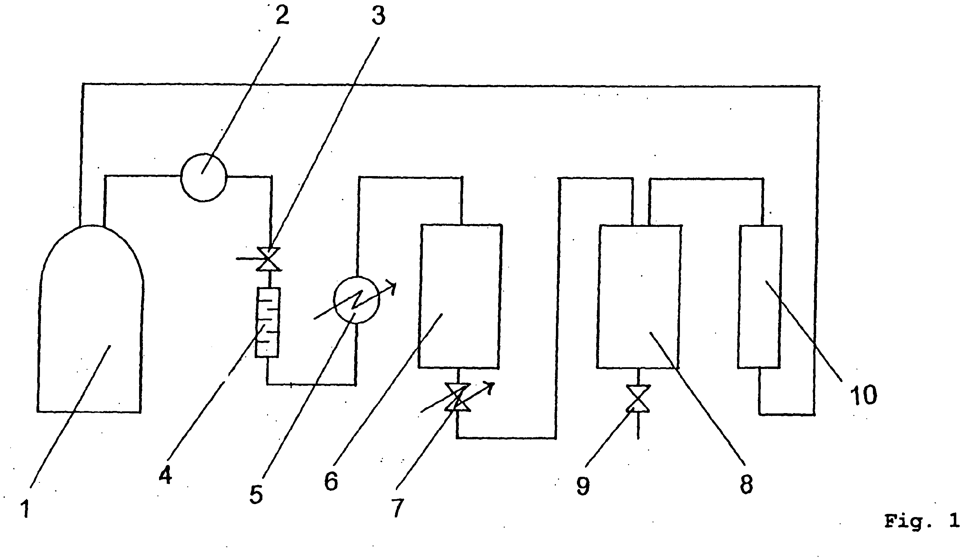

Fig. 1 erläutert schematisch eine Anlage, die zur Durchführung des erfindungsgemäßen Verfahrens geeignet ist.

Aus einem Vorratsbehälter 1 wird CO₂ entnommen und mittels eines Kompressors 2 oder einer Pumpe auf den erforderlichen Druck gebracht. Über eine Injektionsstelle 3 kann ggf. Schleppmittel zugegeben werden, das über eine Mischstrecke 4 mit dem Kohlendioxid vermischt wird. In einem Wärmetauscher 5 wird das Extraktionsmittel auf die gewünschte Temperatur gebracht und anschließend einer Extraktionskammer 6 zugeleitet, wobei der subkritische oder superkritische Zustand erhalten bleibt. Die Extraktionskammer ist mit einem anorganischen Träger, wie Kieselgur, gefüllt, auf welchen das zu extrahierende Pressöl aufgebracht ist. Nach der Extraktion wird der kohlendioxidhaltige Extrakt zur Verringerung des Drucks durch ein beheizbares Druckreduzierventil 7 geleitet und sodann in eine Trennkammer 8 geleitet, wo Extrakt und ggf. Schleppmittel vom Kohlendioxid abgetrennt werden. Der Extrakt kann an der Entnahmestelle 9 aus der Trennkammer 8 entnommen werden. Anschließend wird das Kohlendioxid in einem Kühler 10 gekühlt. Das verflüssigte Gas wird wieder dem Vorratsbehälter 1 zugeführt.

### Beispiel 1: Herstellung des Margosaextrakts

5 kg aus Neemkernen gewonnenes kaltgepresstes Öl werden mit 10 kg Kieselgur (Mischungsverhältnis 1 : 2) vermischt, bis eine homogene Mischung erhalten wird, die frei fließfähig ist. Das als Ausgangsmaterial (AGM) verwendete Pressöl war ein trübes, gold-gelbes Öl. Die Mischung wird in den Extraktionsbehälter einer Extraktionsvorrichtung für Kohlendioxid eingefüllt und dann während 5 Stunden bei 300 bar und 50°C (Druck und Temperatur gemessen am Eingang des Extraktionsgefäßes) extrahiert. Anschließend wurde die Vorlage gewechselt (Fraktion A) und 3 % Ethanol als Schleppmittel zum Kohlendioxid gegeben und für eine weitere Stunde extrahiert. Der Extrakt wurde in einer Vorlage gesammelt (Fraktion B). Der in dem Extrakt der Fraktion B enthaltene Alkohol wurde mit Hilfe eines Rotationsverdampfers in Vakuum abdestilliert und verworfen.

**Tabelle 1: Ausbeuten bei der Kohlendioxidextraktion von 5 kg Neemöl**

| | | |
|---|---|---|
| Ausbeute: | Fraktion A (klares gelbes Öl) | 4,73 kg (94,6 %) |
| | Fraktion B (klares braunes Öl) | 0,27 kg (5,4 %) |

Die Fraktion B wird sehr schnell fest und enthält-vermutlich langkettige Triglyceride.

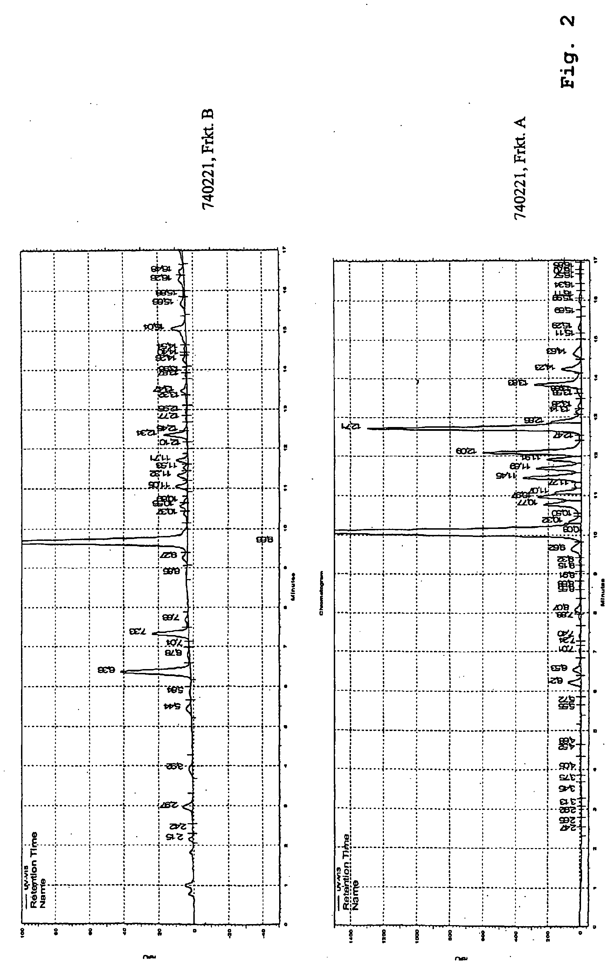

Die Fraktionen A und B sowie das Ausgangsmaterial wurden durch HPLC untersucht. Die Chromatogramme der Fraktionen A und B sind in Fig. 2 dargestellt. Aus den HPLC-Chromatogrammen wurden die Gehalte an Azadirachtin A + B, Nimbin und Salannin bestimmt.

**Tabelle 2: Gehalt an Azadirachtinen, Nimbin und Salannin**

| | % Aza A+B | % Nimbin | % Salannin |
|---|---|---|---|
| Ausgangsmaterial AGM (100%) | 0,22 | 0,28 | 0,78 |
| Fraktion A (94,7%) | 0,24 | 0,27 | 0,74 |
| Fraktion B (5,3%) | 0,0077 | 0,0027 | 0,0068 |

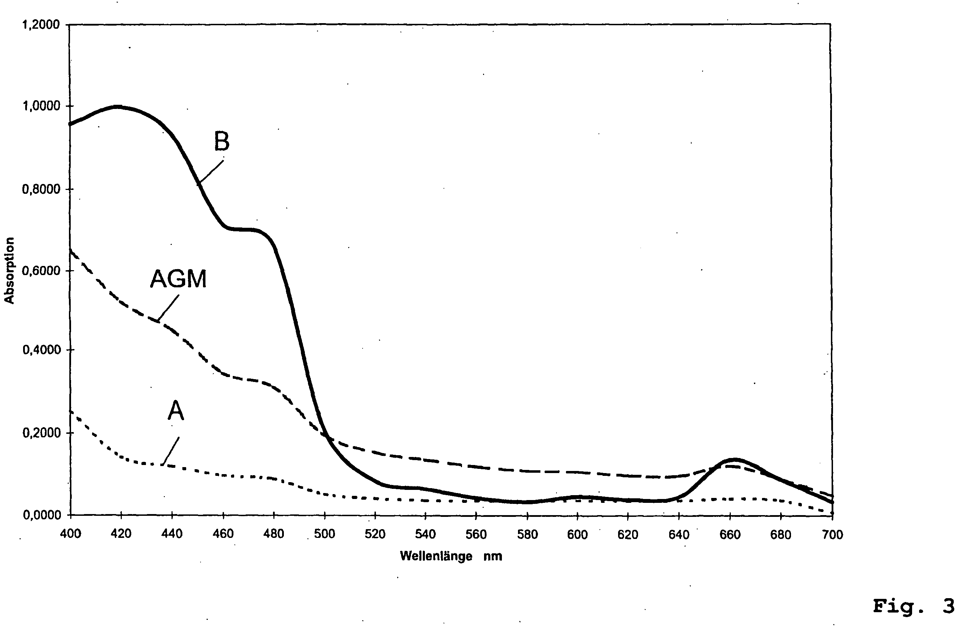

Wie die Werte der Tabelle 2 zeigen, verändert das angewandte Extraktionsverfahren unter Verwendung eines festen mineralischen Trägers die Zusammensetzung der im Ausgangsmaterial vorhandenen biologisch wirksamen Limonoide nicht (Fraktion A). Im Rückstand (Fraktion B) lassen sich nur noch Spuren dieser Verbindungen nachweisen. Es bleibt nur 5,3 % des Ausgangsmaterials im Rückstand, der stark UV-absorbierende Komponenten enthält (s. Fig. 3)

Zur Unterscheidung der verschiedenen Extrakte Pressöl aus Neemsamen, CO₂-Margosaextrakt (Fraktion A) bzw. durch Verwendung eines Schleppmittels gewonnener Margosanachextrakt (Fraktion B)) bieten sich physikalische Parameter an. Zum einen erlaubt ein extrakttypisches HPLC-Chromatogramm den Vergleich der Intensität verschiedener Substanzen. Der Quotient für die Retentionszeiten von 10,02/12,67 Minuten ist beispielsweise für jeden der drei Extrakte signifikant verschieden. Als interner Marker zur Kontrolle der Retentionszeiten wurde das im Handel erhältliche Salannin verwendet. In entsprechender Weise zeigen wellenlängenabhängig unterschiedliche Intensitäten der jeweiligen UV-Absorptionsspektren signifikante Unterschiede (als Beispiel siehe Tabelle 3, Fig. 2 und 3).

**Tabelle 3: Quotienten für Retentionszeiten und UV-Absorption verschiedener Extrakte**

| Quotient | 10,02/12,67 min | 480/660 nm |
|---|---|---|
| Pressöl aus Neemsamenextrakt | 0,16 | 2,86 |
| Margosaextrakt (Fraktion A) | 2,92 | 2,34 |
| Margosanachextrakt (Restöl, Fraktion B) | 12,52 | 5,32 |

### Beispiel 2: Biologische Wirkung

### a) Milbenzucht:

Für die Tests wurde eine Mischkultur aus drei Zuchtansätzen von Dermatophagoides farinae verwendet. Die Milben wurden in Spezialgefäßen auf einem eiweiß- und stärkehaltigen Futter in einem Exsikkator bei Raumtemperatur und bei 80 % relativer Luftfeuchte 30 Tage lang angezogen. Aus diesem Ansatz wurden Milben und Milbeneier in die Testproben eingesetzt.

### b) Prüfsystem

Das Prüfverfahren wurde in Anlehnung an die ISO-Richtlinien AFNOR NF G39-011 durchgeführt. In selbst entwickelten Klimaschränken von 0,055 m³ Inhalt mit durchsichtigen Außenseiten wurde eine kontinuierlich auf und abfahrende Luftfeuchtigkeit von 65 - 80 rF durch eine wässrige Kochsalzlösung eingestellt. Die Temperatur wurde mit Hilfe einer Metallflächenheizung auf 24 °C gehalten. Die Konstanz dieser Bedingungen wurde durch Luftumwälzung und über Sensoren kontrolliert, welche rechnergesteuert überschießende Feuchtigkeits- und Temperaturwerte durch Zufuhr von Frischluft regulierten. Die Konstanz von Temperatur und Feuchtigkeit wurde während der gesamten Versuchszeit registriert.

Es wurden gläserne Petrischalen mit 7 cm Durchmesser und einem speziellen Deckel aus Leichtmetall verwendet, der, mit einem Gummidichtring versehen, die Gasschale luftdicht abschließt. Zur Belüftung der Proben besitzt der Deckel in der Mitte eine runde Öffnung von 3 cm Durchmesser, die mit einer Lüftungsmembran aus Polytetrafluorethylen (PTFE) verschlossen ist. Als Substrat für die Milben wurde eine Mischung aus milbenfreiem Hausstaub und ca. 0,25 g Futter pro Schale zugesetzt. In die Schalen wurden jeweils etwa 200 Milben und 50 Eier eingebracht. Maximal 18 Proben wurden pro Klimaschrank eingestellt.

Die verschiedenen Prüfsubstanzen wurden in die vorbereiteten, mit Futter und Milben 12 Stunden lang vorinkubierten Gefäße in einer Menge von jeweils 20 ml/m² möglichst gleichmäßig eingesprüht. Von jeder Probe wurden vier bis sechs Schalen, von der Isopropylalkohol-Kontrolle ebenfalls sechs Schalen angesetzt. Dann wurde zur Kontrolle der Kot- und Allergenproduktion im Deckel jeder Schale eine runde Haftscheibe aus Kunststoff (Dicke 0,01 mm, Durchmesser 3 cm) mit Hilfe eines Klebstreifens so angeklebt, dass sie den Boden berührte und die Milben darüber laufen konnten. Nach Verdampfen des Alkohols wurden die versuchsgefäße in den Klimaschrank gestellt und wöchentlich auf Mortalität sowie Änderung des Verhaltens der Milben geprüft.

Nach 14 Wochen wurde der Versuch beendet. Die Länge der Versuchsdauer erlaubt auch eine Aussage über die Wirkung der Prüfsubstanzen auf die Fertilität der Eier. Bei den wöchentlichen Kontrollen wurde in keinem Fall festgestellt, dass alle Versuchstiere gestorben waren.

Als Prüfsubstanzen wurden die folgenden Proben verwendet:

| | | |
|---|---|---|
| Kontrolle | (1) | Isopropanol |
| Margosaöl | (2) | Extrakt aus Neemöl, erhalten in Beispiel 1 |
| Neemöl | (3) | Extrakt aus Neemsamen, hergestellt nach der WO 97/25867, erhalten durch direkte Extraktion gemahlener Neemnüsse mit CO₂ |

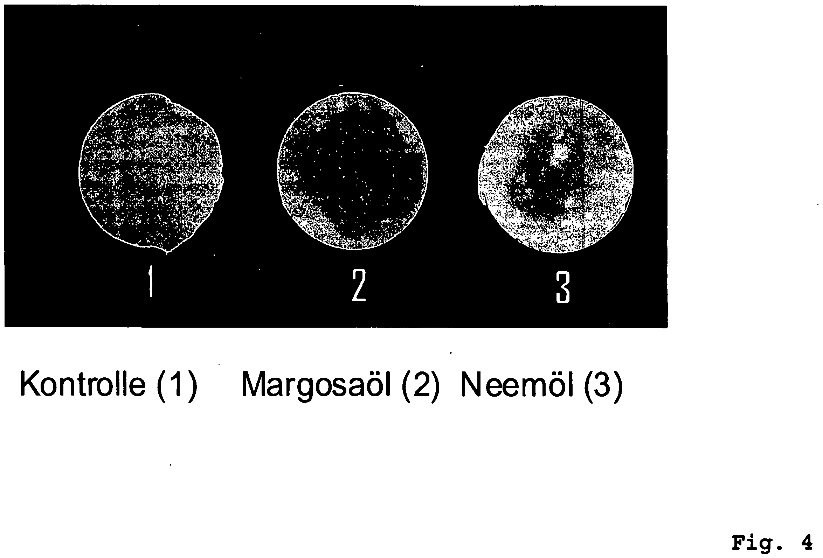

In Figur 4 sind die runden Haftstreifen aus Kunststoff abgebildet, die beim oben geschilderten Versuchsablauf in den Testgefäßen positioniert waren. Die Haftstreifen sind ursprünglich transparent und für die Darstellung auf einem schwarzen Untergrund aufgelegt. Der Milbenkot entspricht den weißen Bereichen, während in Abschnitten die nicht oder nur wenig von Milbenkot bedeckt sind dunkel erscheinen, da der schwarze Untergrund durch die transparenten Haftscheiben sichtbar bleibt. Das Ausmaß der hellen Flächen gibt die Größe der Milbenpopulation wieder, da eine große Milbenpopulation auch große Mengen an Milbenkot produziert. Je größer die dunklen Flächen ausfallen, umso kleiner ist die Milbenpopulation.

Bei der Kontrolle (1) hat sich auf der Haftscheibe eine dicke weiße Schicht aus Milbenkot abgelagert. Der schwarze Untergrund wird vom Milbenkot dicht abgedeckt. Das Wachstum der Milben ist nicht gehemmt.

Beim erfindungsgemäßen Margosaextrakt (2) sind lediglich am Rand der Haftscheibe weiße Flächen zu erkennen, die eine Bedeckung mit Milbenkot anzeigen, während der innere Abschnitt der Haftscheibe weitgehend von Milbenkot unbedeckt ist und daher dunkel erscheint.

Bei Extrakt aus Neemsamen (3), der durch direkte Extraktion der gemahlenen Neemsamen mit Kohlendioxid erhalten wurde, ist ein breiterer weißer Rand zu erkennen. Im Zentrum der Haftscheibe scheint jedoch der dunkle Untergrund durch, sodass auch bei diesem Extrakt eine deutliche Hemmung des Wachstums der Milbenpopulation beobachtet wird, wenn auch nicht in einem Ausmaß, wie er beim Margosaextrakt erreicht wird. Die Kotmenge ist jedoch deutlich niedriger als bei der Kontrolle (1).

Durch den Margosaextrakt (2) wie auch durch den Extrakt aus Neemsamen wird erreicht, dass die Milben die Nahrungsaufnahme weitgehend verweigern und schließlich verhungern, ohne sich vorher fortzupflanzen. Die Milbenpopulation und damit auch das im Kot enthaltene Milbenallergen nehmen daher mit der Zeit immer weiter ab. Die Wirkung ist beim erfindungsgemäßen Margosaextrakt gegenüber dem Extrakt aus Neemsamen jedoch noch einmal deutlich erhöht.

## Patentansprüche

1. Verfahren zur Herstellung eines Margosaextraktes, wobei ein Öl durch Auspressen aus Kernen und/oder Samen des Neembaums gewonnen wird und das Öl bei einem Druck von 250 bis 600 bar mit einem Kohlendioxid enthaltenden Extraktionsmittel extrahiert wird.

2. Verfahren nach Anspruch 1, wobei das Öl auf einen inerten Träger aufgebracht wird.

3. Verfahren nach Anspruch 2, wobei als Träger ein pulverförmiges anorganisches Material verwendet wird.

4. Verfahren nach Anspruch 2 oder 3, wobei das anorganische Material ausgewählt ist aus Kieselgur und Kieselgel.

5. Verfahren nach einem der vorhergehenden Ansprüche, wobei die Extraktion bei einem Druck von 300 bis 400 bar durchgeführt wird.

6. Verfahren nach einem der vorhergehenden Ansprüche, wobei die Extraktion bei einer Temperatur im Bereich von 10 bis 70 °C durchgeführt wird.

7. Verfahren nach einem der vorhergehenden Ansprüche, wobei das Extraktionsmittel im Kreislauf geführt wird.

8. Verfahren nach einem der vorhergehenden Ansprüche, wobei das Extraktionsmittel bei einer Temperatur von 10 bis 80 °C, vorzugsweise 25 bis 40 °C auf einen Druck von 30 bis 80 bar, vorzugsweise 50 bis 70 bar entspannt wird.

9. Verfahren nach einem der vorhergehenden Ansprüche, wobei das Extraktionsmittel einen Anteil eines Schleppmittels enthält.

10. Verfahren nach einem der vorhergehenden Ansprüche, wobei die Extraktion fraktioniert durchgeführt wird.

11. Margosaextrakt, erhältlich mit einem Verfahren gemäß einem der Ansprüche 1 bis 10.

12. Verwendung eines Margosaextraktes nach Anspruch 11 zur Schädlingsbekämpfung.

13. Verwendung nach Anspruch 12, als Repellens gegen die Hausstaubmilbe.

14. Schädlingsbekämpfungsmittel, enthaltend einen Margosaextrakt nach Anspruch 11.

## Claims

1. Method for producing a margosa extract, wherein an oil is obtained by pressing from kernels and/or seeds of the neem tree, and the oil is extracted at a pressure of from 250 to 600 bar with an extracting agent containing carbon dioxide.

2. Method according to claim 1, wherein the oil is applied to an inert carrier.

3. Method according to claim 2, wherein a pulverant inorganic material is used as carrier.

4. Method according to claim 2 or 3, wherein the inorganic material is selected from diatomaceous earth and silica gel.

5. Method according to one of the previous claims, wherein the extraction is carried out at a pressure of from 300 to 400 bar.

6. Method according to one of the previous claims, wherein the extraction is carried out at a temperature in the range of from 10 to 70°C.

7. Method according to one of the previous claims, wherein the extracting agent is recycled.

8. Method according to one of the previous claims, wherein the extracting agent is depressurized at a temperature of 10 to 80°C, preferably 25 to 40°C, to a pressure of from 30 to 80 bar, preferably 50 to 70 bar.

9. Method according to one of the previous claims, wherein the extracting agent contains a portion of an entrainer.

10. Method according to one of the previous claims, wherein the extraction is carried out fractionated.

11. Margosa extract, which can be obtained with a method according to one of claims 1 to 10.

12. Use of a margosa extract according to claim 11 for pest control.

13. Use according to claim 12, as repellent against the house dust mite.

14. Pesticide, containing a margosa extract according to claim 11.

## Revendications

1. Procédé de préparation d'un extrait de margosa, consistant à presser les noyaux et/ou graines du margousier pour obtenir une huile et à extraire ladite huile, à une pression de 250 à 600 bar, avec un agent d'extraction contenant du dioxyde de carbone.

2. Procédé selon la revendication, ladite huile étant appliquée sur un support inerte.

3. Procédé selon la revendication 2, un matériau inorganique pulvérulent étant utilisé en tant que support.

4. Procédé selon la revendication 2 ou 3, ledit matériau inorganique étant choisi parmi le kieselguhr et le gel de silice.

5. Procédé selon l'une des revendications précédentes, ladite extraction étant réalisée à une pression de 300 à 400 bar.

6. Procédé selon l'une des revendications précédentes, ladite extraction étant réalisée à une température comprise entre de 10 à 70 °C.

7. Procédé selon l'une des revendications précédentes, ledit agent d'extraction étant recyclé.

8. Procédé selon l'une des revendications précédentes, ledit agent d'extraction étant détendu, à une température de 10 à 80 °C, préférentiellement de 25 à 40 °C, pour atteindre une pression de 30 à 80 bar, préférentiellement de 50 à 70 bar.

9. Procédé selon l'une des revendications précédentes, ledit agent d'extraction contenant une proportion d'un agent d'entraînement.

10. Procédé selon l'une des revendications précédentes, ladite extraction étant réalisée de façon fractionnée.

11. Extrait de margosa, pouvant être obtenu avec un procédé selon l'une des revendications 1 à 10.

12. Utilisation d'un extrait de margosa selon la revendication 11 pour lutter contre les nuisibles.

13. Utilisation selon la revendication 12, en tant que répulsif contre les acariens domestiques.

14. Pesticide, contenant un extrait de margosa selon la revendication 11.
